# EUROPEAN PATENT APPLICATION

(11) **EP 4 715 045 A1**
(43) Date of publication of application: **25.03.2026**
(21) Application number: 24811348.2
(22) Date of filing: 17.05.2024
(51) Int. Cl.: C12N 9/10, C12N 15/77, C12P 13/08

(54) **MUTANT POLYPEPTIDE AND METHOD FOR PRODUCING L-VALINE USING SAME**

(30) Priority: 19.05.2023 KR 20230065142
(71) Applicant: CJ Cheiljedang Corporation, Seoul 04560 (KR)
(72) Inventor: KIM, Seon Hye, Seoul 04560 (KR); CHO, Hyun Kug, Seoul 04560 (KR); YUN, Hyojin, Seoul 04560 (KR); LEE, Hyein, Seoul 04560 (KR); KIM, So-Yeon, Seoul 04560 (KR); OH, Haena, Seoul 04560 (KR); JU, Si Yeon, Seoul 04560 (KR); LEE, Jungseok, Seoul 04560 (KR); KIM, Sang Jun, Seoul 04560 (KR); CHOI, Jin-Geun, Seoul 04560 (KR)
(74) Representative: Meissner Bolte Partnerschaft mbB
(86) International application number: PCT/KR2024/006708
(87) International publication number: WO 2024/242409

(57) **Abstract**

The present disclosure relates to: a variant polypeptide; a polynucleotide encoding the variant polypeptide; a microorganism comprising the variant polypeptide or a polynucleotide encoding the variant polypeptide; a method for producing L-valine, comprising culturing the microorganism in a medium; and a method for increasing the L-valine producing ability of a microorganism.

## Description

### [Technical Field]

The present disclosure relates to: a variant polypeptide; a polynucleotide encoding the variant polypeptide; a microorganism comprising the variant polypeptide or a polynucleotide encoding the variant polypeptide; use for L-valine production; a method for producing L-valine; and a method for increasing the L-valine producing ability of a microorganism.

### [Background Art]

L-Amino acids are the fundamental building blocks of proteins and are used as important materials for pharmaceutical raw materials, food additives, animal feed, nutritional supplements, insecticides, and fungicides. In particular, branched-chain amino acids (BCAAs), which collectively refer to the essential amino acids L-valine, L-leucine, and L-isoleucine, have antioxidant effects and promote protein synthesis in muscle cells.

BCAA production using microorganisms is mainly achieved through microorganisms of the genus *Corynebacterium,* and is known to biosynthesize them from pyruvic acid through multiple steps using 2-ketoisocaproate as a precursor [Korean Patent No. 10-0220018 and Korean Patent No. 10-0438146]. However, BCAA production through such microorganisms has a problem in that large-scale industrial manufacturing cannot be easily achieved.

### [Disclosure]

### [Technical Problem]

The present disclosure relates to: a variant polypeptide; a polynucleotide encoding the variant polypeptide; a microorganism comprising the variant polypeptide or a polynucleotide encoding the variant polypeptide; use for L-valine production; a method for producing L-valine; and a method for increasing the L-valine producing ability of a microorganism.

### [Technical Solution]

An aspect of the present disclosure provides a variant polypeptide in which an amino acid corresponding to position 154 in SEQ ID NO: 1 is substituted with a different amino acid.

In one specific embodiment, the amino acid corresponding to position 154 in SEQ ID NO: 1 may be substituted with an electrically charged amino acid.

In one specific embodiment of the foregoing embodiment, the electrically charged amino acid may be arginine, histidine, or glutamic acid.

In another specific embodiment, the variant polypeptide may comprise any one amino acid sequence from the amino acid sequences represented by Formula 1 to Formula 3 below:
[Formula 1]
   EPFGIRELIX
[Formula 2]
   PGKLRALLDVLEPFGIRELIX
[Formula 3] 
GINRITVVVDADELNIEQITKQLNKLIPVLKVVRLDEETTIARAIMLVKVSADST
NRPQIVDAANIFRARVVDVAPDSVVIESTGTPGKLRALLDVMEPFGIRELIX
 wherein in Formulas 1 to 3 above, X may be an electrically charged amino acid.

In a variant polypeptide according to any one of the foregoing specific embodiments, the X may be arginine, histidine, or glutamic acid.

In another specific embodiment, the variant polypeptide may be a polypeptide in which the amino acid corresponding to position 42 in the amino acid sequence of SEQ ID NO: 1 is additionally substituted with a different amino acid.

In a variant polypeptide according to any one of the foregoing specific embodiments, the different amino acid may be valine.

In a variant polypeptide according to any one of the foregoing specific embodiments, the variant polypeptide may comprise an amino acid sequence of NLVSLVSV, LSVLVQDVDGIISRVSGMFTRRAFNLVSLVSV, or MANSDVTRHILSVLVQDVDGIISRVSGMFTRRAFNLVSLVSV.

In another specific embodiment, the variant polypeptide may comprise an amino acid sequence having 70% or more and less than 100% identity to SEQ ID NO: 1.

In another specific embodiment, the variant polypeptide may be a variant of an acetohydroxy acid synthase subunit.

In another specific embodiment, the variant polypeptide may comprise an amino acid sequence of SEQ ID NO: 3, SEQ ID NO: 5, SEQ ID NO: 7, SEQ ID NO: 8, SEQ ID NO: 9, or SEQ ID NO: 10.

Another aspect of the present disclosure provides a polynucleotide encoding the variant polypeptide.

Still another aspect of the present disclosure provides a microorganism comprising the variant polypeptide or a polynucleotide encoding the variant polypeptide.

In one specific embodiment, the microorganism may have increased L-valine-producing ability compared to a microorganism comprising a polypeptide of SEQ ID NO: 1 or a polynucleotide encoding the same.

In a microorganism according to any one of the foregoing specific embodiments, the microorganism may be a microorganism of the genus *Corynebacterium.*

In a microorganism according to any one of the foregoing specific embodiments, the microorganism of the genus *Corynebacterium* may be *Corynebacterium glutamicum.*

Still another aspect of the present disclosure provides a method for producing L-valine, comprising culturing the microorganism in a medium.

In one specific embodiment, the method may further comprise recovering L-valine from the cultured microorganism, the culture of the microorganism, the fermentation product of the microorganism, or the culture medium.

Still another aspect of the present disclosure provides a composition for producing L-valine, comprising: the variant polypeptide; a polynucleotide encoding the variant polypeptide; a vector comprising the polynucleotide; a microorganism comprising the variant polypeptide, a polynucleotide encoding the variant polypeptide, or a vector comprising the polynucleotide; a culture of the microorganism; or a combination of two or more thereof.

Still another aspect of the present disclosure provides use of the variant polypeptide or a microorganism comprising the variant polypeptide or a polynucleotide encoding the variant polypeptide for L-valine production.

Still another aspect of the present disclosure provides a method for increasing the L-valine producing ability of a microorganism, comprising modifying the microorganism to express the variant polypeptide.

### [Advantageous Effects]

Culturing a microorganism comprising the variant polypeptide of the present disclosure enables L-valine production at high yield compared to a microorganism comprising a conventional, non-modified polypeptide.

### [Detailed Description of Preferred Embodiments]

The present disclosure will be described in detail as follows. Meanwhile, each description and embodiment disclosed herein can be applied to other descriptions and embodiments, respectively. That is, all combinations of various elements described herein fall within the scope of the present invention. Additionally, the scope of the present disclosure is not limited by the specific description described below. Additionally, a number of papers and patent documents have been cited throughout the present specification. The content of the cited papers and patent documents is incorporated herein by reference in their entirety to describe the level of the technical field to which the present disclosure belongs and the contents of the present disclosure more clearly.

An aspect of the present disclosure provides a variant polypeptide in which an amino acid corresponding to position 154 in SEQ ID NO: 1 is substituted with a different amino acid.

As used herein, the term "variant" or "protein variant" refers to a polypeptide in which conservative substitution or modification of one or more amino acids is present, such that it differs from a parent sequence of the polypeptide while maintaining the functions or properties of the polypeptide. The variant polypeptide differs from an identified sequence by multiple amino acid substitutions, deletions, or additions. Such a variant polypeptide may typically be identified by modifying one or more amino acids in the amino acid sequence of the polypeptide and evaluating the properties of the modified polypeptide. That is, the ability of the variant polypeptide may be enhanced, unchanged, or decreased compared to the ability of the natural polypeptide. Additionally, certain variant polypeptides may include a variant polypeptide in which one or more regions, such as an N-terminal leader sequence or transmembrane domain, have been removed. Other variant polypeptides may include a variant in which a portion is removed from the N- and/or C-terminus of the mature protein. The term "variant polypeptide" may be used interchangeably with terms such as modification, modified polypeptide, modified protein, mutant, mutein, and divergent, and is not limited thereto as long as it is a term used in a mutated sense.

Additionally, the variant polypeptide may include deletions or additions of amino acids that have a minimal effect on the properties and secondary structure of the polypeptide. For example, a polypeptide may be conjugated with a signal (or leader) sequence at the N-terminus of a polypeptide involved in the translocation of a protein co-translationally or post-translationally. Additionally, the polypeptide may also be conjugated with another sequence or linker to allow for identification, purification, or synthesis of a polypeptide.

For example, the variant polypeptide disclosed herein refers to a polypeptide comprising one or more amino acid substitutions in the amino acid sequence of a wild-type polypeptide, or a polypeptide comprising one or more amino acid substitutions in the amino acid sequence [parent sequence] of a known polypeptide.

As used herein, the term "wild-type" refers to a naturally-occurring state without artificial modification. When the term "wild-type" is used in reference to a polypeptide, it refers to a naturally-occurring polypeptide which does not have artificial mutations (substitutions, insertions, deletions, *etc.)* at one or more amino acid positions. Similarly, when the term "wild-type" is used in reference to a polynucleotide, it refers to a naturally occurring polynucleotide which does not have artificial modifications (substitutions, insertions, deletions, *etc.)* at one or more nucleotide positions. However, a polynucleotide encoding a wild-type polypeptide is not limited to natural polynucleotides and includes sequences encoding any wild-type polypeptide.

As used herein, the term "parent sequence" or "backbone" refers to a reference sequence into which modifications are introduced to create a variant polypeptide. That is, the parent sequence may be a starting sequence into which modifications such as substitutions, additions, and/or deletions are to be introduced. The parent sequence may be a naturally-occurring or wild-type sequence, a variant in which one or more substitutions, insertions, deletions, *etc.* have occurred in the naturally-occurring or wild type sequence, or an artificially synthesized sequence.

As used herein, the polypeptide comprising a parent sequence may be a polypeptide with acetohydroxy acid synthase subunit activity or IIvN. For example, the sequence of the polypeptide with acetohydroxy acid synthase subunit activity of the present disclosure may be obtained from GenBank of NCBI, a known database. In one example, the polypeptide with acetohydroxy acid synthase subunit activity of the present disclosure may be a polypeptide derived from a microorganism of the genus *Corynebacterium* or from the *Corynebacterium glutamicum* strain, but is not limited thereto.

As used herein, the term "acetohydroxy acid synthase", which is also referred to as acetolactate synthase, refers to the first enzyme used in the biosynthesis of L-valine. Acetohydroxy acid synthase can catalyze the decarboxylation of pyruvate and a condensation reaction with another pyruvate molecule to produce acetolactate, a precursor of valine, or catalyze the decarboxylation of pyruvate and a condensation reaction with 2-ketobutyrate to produce acetohydroxybutyrate, a precursor of isoleucine.

Acetohydroxy acid synthase is encoded by two genes, *ilvB* and *ilvN.* The *ilvB* gene encodes the large subunit of acetohydroxy acid synthase, and the *ilvN* gene encodes the small subunit of acetohydroxy acid synthase. Among the two subunits, the small subunit encoded by the *ilvN* gene is thought to be crucial for feedback inhibition.

As used herein, the term "acetohydroxy acid synthase subunit" is not particularly limited but may be an acetohydroxy acid synthase subunit encoded by the *ilvN* gene or an IIvN protein.

In one example, the polypeptide comprising a parent sequence of the present disclosure may comprise the amino acid sequence of SEQ ID NO: 1 or an amino acid sequence having 70% or more homology or identity thereto, but is not limited thereto as long as it retains acetohydroxy acid synthase subunit activity. Specifically, a protein with the amino acid sequence of SEQ ID NO: 1, in which part of the sequence is deleted or substituted, or into which a different sequence is added, may be included in the polypeptide comprising the parent sequence, as long as the protein exhibits an efficacy corresponding to the acetohydroxy acid synthase subunit activity. Specifically, the polypeptide comprising a parent sequence may comprise the amino acid sequence of SEQ ID NO: 1 or an amino acid sequence having at least 70%, 80%, 85%, 90%, 95%, 96%, 97%, 98%, or 99% or more homology or identity to the amino acid sequence of SEQ ID NO: 1. The sequence of SEQ ID NO: 1 can be obtained from GenBank of NCBI or KEGG(Kyoto Encyclopedia of Genes and Genomes), which are known databases. In one example, the polypeptide comprising the parent sequence may be derived from a microorganism of the genus *Corynebacterium* or *Corynebacterium glutamicum,* and more specifically, may be a polypeptide/protein comprising the amino acid sequence set forth in SEQ ID NO: 1, but is not limited thereto. Additionally, it is apparent that an auxiliary protein having an amino acid sequence in which part of the sequence is deleted, modified, substituted, or added is also included within the scope of the present disclosure, as long as the amino acid sequence has such homology or identity and exhibits equivalent efficacy to that of the protein.

Meanwhile, the expression "the amino acid corresponding to a specific position of (in) a specific amino acid sequence is substituted with a different amino acid" used herein is understood as that the amino acid sequence is used as a reference sequence. In particular, the parent sequence may have any structure as long as it is not particularly defined. Examples of the parent sequence are as described above.

As used herein, the term "reference sequence" refers to a sequence used to determine the Nth position within a specific amino acid sequence. For example, when a specific amino acid sequence and a reference sequence are aligned using a sequence alignment known in the art and numbering each amino acid residue in the specific amino acid sequence with reference to the amino acid residue position of the reference sequence based thereon, the amino acid position in the specific amino acid sequence corresponding to the N^{th} position of the reference sequence can be determined.

Specifically, the sequence of a polynucleotide encoding a polypeptide comprising a parent sequence having the amino acid sequence of SEQ ID NO: 1 or an amino acid sequence having 70% or more homology or identity to SEQ ID NO: 1 can be obtained, for example, based on codon information known in the art. In one example, the polypeptide comprising a parent sequence may be encoded by a polynucleotide having, comprising, consisting of, or essentially consisting of the sequence of SEQ ID NO: 2 or a base sequence having 70% or more, 75% or more, 80% or more, 85% or more, 90% or more, 95% or more, 96% or more, 97% or more, 98% or more, or 99% or more homology or identity to the sequence of SEQ ID NO: 2, but is not limited thereto. Additionally, the base sequence of SEQ ID NO: 2 can be obtained from a known database, such as GenBank of NCBI, but is not limited thereto.

In the present disclosure, "polynucleotide (gene) comprising the base sequence of SEQ ID NO: 2" can be used interchangeably with "polynucleotide (gene) having the base sequence of SEQ ID NO: 2" or "polynucleotide (gene) consisting of the base sequence of SEQ ID NO: 2".

The variant polypeptide of the present disclosure may be a variant polypeptide in which an amino acid corresponding to position 154 in SEQ ID NO: 1 is substituted with a different amino acid.

In one embodiment, the variant polypeptide of the present disclosure may have 70% or more and less than 100% sequence homology to SEQ ID NO: 1, but is not limited thereto.

Specifically, the variant of the present disclosure may comprise, in an amino acid sequence having at least 70%, 75%, 80%, 85%, 90%, 95%, 96%, 97%, 98%, 99% or more and less than 100% homology or identity to the amino acid sequence set forth in SEQ ID NO: 1, substitution of the amino acid at position 154 from the N-terminus of SEQ ID NO: 1 with a different amino acid. Additionally, it is apparent that a variant having an amino acid sequence in which part of the sequence is deleted, modified, substituted, conservatively substituted, or added is also included within the scope of the present disclosure, as long as the amino acid sequence has such homology or identity and exhibits equivalent efficacy to that of the variant of the present disclosure.

In one embodiment, the variant polypeptide may be a variant of an acetohydroxy acid synthase subunit, but is not limited thereto.

The "different amino acid" is not limited as long as the amino acid differs from the amino acid before substitution. Meanwhile, when the expression "a specific amino acid is substituted" is used herein, it is apparent that the amino acid is substituted with an amino acid different from the amino acid before substitution, even if the present disclosure does not particularly specify that the substitution is with a different amino acid.

Amino acids can generally be classified based on similarity in polarity, charge, solubility, hydrophobicity, hydrophilicity, and/or amphipathic nature of the residues.

For example, positively charged (basic) amino acids include arginine, lysine, and histidine; negatively charged (acidic) amino acids include glutamic acid and aspartate; amino acids with nonpolar side chains (nonpolar amino acids) include glycine, alanine, valine, leucine, isoleucine, methionine, phenylalanine, tryptophan, and proline; and amino acids with polar or hydrophilic side chains (polar amino acids) include serine, threonine, cysteine, tyrosine, asparagine, and glutamine. In another example, electrically charged amino acids include arginine, lysine, histidine, glutamic acid, and aspartate, and amino acids with electrically uncharged side chains (also referred to as uncharged amino acids or neutral amino acids) include glycine, alanine, valine, leucine, isoleucine, methionine, phenylalanine, tryptophan, proline, serine, threonine, cysteine, tyrosine, asparagine, and glutamine. In another example, phenylalanine, tryptophan, and tyrosine may be classified as aromatic amino acids. In another example, valine, leucine, and isoleucine may be classified as branched-chain amino acids (branched amino acids). In another example, 20 types of amino acids may be classified into five groups by size, starting from the group of amino acids with relatively small volumes: glycine, alanine, and serine; cysteine, proline, threonine, aspartate, and asparagine; valine, histidine, glutamic acid, and glutamine; isoleucine, leucine, methionine, leucine, and arginine; and phenylalanine, tryptophan, and tyrosine. However, the classification of amino acids is not limited thereto.

For example, when the phrase "the amino acid corresponding to position 154 in SEQ ID NO: 1 is substituted with a different amino acid" is used, it may mean that the amino acid is substituted with arginine, histidine, glutamic acid, valine, glycine, isoleucine, phenylalanine, aspartate, cysteine, asparagine, alanine, proline, serine, tyrosine, lysine, tryptophan, methionine, threonine, or leucine, excluding glutamine, but is not limited thereto.

Even when the expression "a protein having the amino acid sequence set forth in a specific SEQ ID NO" is used in the present disclosure, it is apparent that a protein having an amino acid sequence in which part of the sequence is deleted, modified, substituted, conservatively substituted, or added may also be used in the present disclosure, as long as the protein exhibits identical or equivalent activity to that of a protein consisting of the amino acid sequence of the corresponding SEQ ID NO. For example, as long as it exhibits identical or equivalent activity to that of the variant protein, it is apparent that a protein with sequence additions, naturally-occurring mutations, or silent mutations or conservative substitutions thereof that do not alter the protein function before or after the amino acid sequence is not excluded, and that such protein with sequence additions or mutations falls within the scope of the present disclosure.

The term "position N" of the present disclosure may include the Nth position and the amino acid positions corresponding to the N^{th} position. Specifically, the term may include amino acid positions corresponding to any amino acid residues in a mature polypeptide represented by a specific amino acid sequence. The specific amino acid sequence may be the amino acid sequence of SEQ ID NO: 1.

As used herein, the term "corresponding to" refers to an amino acid residue at a specific position in a polypeptide or an amino acid residue that is similar, identical, or homologous to an amino acid residue at the specific position in a polypeptide. Identification of the amino acid at the corresponding position may be determination of a specific amino acid in a sequence that references a particular sequence. As used herein, the term "corresponding region" refers to a similar or corresponding position in a related protein or a reference protein.

For example, any amino acid sequence can be aligned with SEQ ID NO: 1, and based thereon, each amino acid residue in the amino acid sequence can be numbered with reference to the position number of the amino acid residue corresponding to the amino acid residue of SEQ ID NO: 1. For example, a sequence alignment algorithm as described herein can identify the position of an amino acid or a position where modification such as substitution, insertion, or deletion occurs compared to a query sequence (also referred to as the "reference sequence").

For such an alignment, for example, the Needleman-Wunsch algorithm (Needleman and Wunsch, 1970, J. Mol. Biol. 48: 443-453), the Needleman program of the EMBOSS package (EMBOSS: The European Molecular Biology Open Software Suite, Rice et al., (2000), Trends Genet. 16: 276-277), and the like may be used, but the program is not limited thereto. Any sequence alignment program known in the art, such as a pairwise sequence comparison algorithm, may be used as appropriate.

In one embodiment of the foregoing embodiments, the variant polypeptide of the present disclosure may include substitution of the amino acid corresponding to position 154 in SEQ ID NO: 1 with an electrically charged amino acid, specifically arginine, histidine, glutamic acid, lysine, or aspartate, but is not limited thereto.

In one embodiment of the foregoing embodiments, the variant polypeptide of the present disclosure may be a polypeptide in which the amino acid corresponding to position 154 in SEQ ID NO: 1 is substituted with arginine, histidine, or glutamic acid, but is not limited thereto.

For example, the variant polypeptide of the present disclosure may comprise an amino acid sequence, in which the amino acid corresponding to position 154 in the amino acid sequence set forth in SEQ ID NO: 1 is fixed to arginine, histidine, or glutamic acid, and in which the sequence has at least 70%, 75%, 80%, 85%, 90%, 95%, 96%, 97%, 98%, or 99% or more and less than 100% homology or identity to SEQ ID NO: 1. Additionally, it is apparent that a variant polypeptide having an amino acid sequence in which part of the sequence is deleted, modified, substituted, conservatively substituted, or added is also included within the scope of the present disclosure, as long as the amino acid sequence has such homology or identity and exhibits equivalent efficacy to that of the variant polypeptide of the present disclosure.

Meanwhile, those skilled in the art can identify the amino acid in any amino acid sequence that corresponds to position 154 in the amino acid sequence of SEQ ID NO: 1 of the present disclosure through a sequence alignment known in the art. Additionally, it is apparent that when the expression "an amino acid at a specific position in a specific SEQ ID NO" is used, it also includes "the amino acid corresponding to the specific position" in any amino acid sequence even without a particular description in the present disclosure.

In another specific embodiment, the variant polypeptide may comprise any one amino acid sequence from the amino acid sequences represented by Formula 1 to Formula 3 below.
[Formula 1]
   EPFGIRELIX
[Formula 2]
   PGKLRALLDVLEPFGIRELIX
[Formula 3]

GINRITVVVDADELNIEQITKQLNKLIPVLKVVRLDEETTIARAIMLVKVSADST
NRPQIVDAANIFRARVVDVAPDSVVIESTGTPGKLRALLDVMEPFGIRELIX

In Formula 1 to Formula 3 above, X may be an electrically charged amino acid.

Specifically, in Formula 1 to Formula 3 above, the X may be an amino acid corresponding to the 154^{th} position in the amino acid sequence of SEQ ID NO: 1 of the present disclosure.

In a variant polypeptide according to any one of the foregoing specific embodiments, the X may be arginine, histidine, or glutamic acid.

In one example, Formula 1 above may consist of SEQ ID NO: 23, Formula 2 above may consist of SEQ ID NO: 24, and Formula 3 above may consist of SEQ ID NO: 25.

In one specific embodiment, the variant polypeptide of the present disclosure may further comprise substitution of the amino acid corresponding to position 42 in the amino acid sequence of SEQ ID NO: 1 with a different amino acid, in addition to the substitution of the amino acid corresponding to position 154 in an amino acid sequence of SEQ ID NO: 1 with a different amino acid.

In one embodiment, the variant polypeptide of the present disclosure may include an amino acid sequence in which one or two of the amino acids corresponding to positions 42 and 154 from the N-terminus in SEQ ID NO: 1 are substituted with different amino acids.

In one embodiment, the variant polypeptide of the present disclosure may be a polypeptide in which the amino acid corresponding to position 42 in SEQ ID NO: 1 is substituted with valine, but is not limited thereto.

In one embodiment of the foregoing embodiments, the variant polypeptide of the present disclosure may be a polypeptide in which the amino acid corresponding to position 154 in SEQ ID NO: 1 is substituted with arginine, histidine, or glutamic acid and in which the amino acid corresponding to position 42 is substituted with valine, but is not limited thereto.

For example, the variant polypeptide of the present disclosure may comprise an amino acid sequence in which the amino acid corresponding to position 154 based on the amino acid sequence set forth in SEQ ID NO: 1 is fixed to arginine, histidine, or glutamic acid and the amino acid corresponding to position 42 is fixed to valine, and in which the amino acid sequence has 70%, 75%, 80%, 85%, 90%, 95%, 96%, 97%, 98%, or 99% or more and less than 100% homology or identity to the amino acid sequence of SEQ ID NO: 1. Additionally, it is apparent that a variant polypeptide having an amino acid sequence in which part of the sequence is deleted, modified, substituted, conservatively substituted, or added is also included within the scope of the present disclosure, as long as the amino acid sequence has such homology or identity and exhibits equivalent efficacy to that of the variant polypeptide of the present disclosure.

In a variant polypeptide according to any one of the foregoing specific embodiments, the variant polypeptide may comprise an amino acid sequence of NLVSLVSV (SEQ ID NO: 26), LSVLVQDVDGIISRVSGMFTRRAFNLVSLVSV (SEQ ID NO: 27), or MANSDVTRHILSVLVQDVDGIISRVSGMFTRRAFNLVSLVSV (SEQ ID NO: 28).

Specifically, the amino acid valine positioned at the C-terminus of the amino acid sequence of SEQ ID NO: 26 to SEQ ID NO: 28 may be an amino acid corresponding to position 42 in the amino acid sequence of SEQ ID NO: 1 of the present disclosure.

In another specific embodiment, the variant polypeptide may comprise an amino acid sequence of SEQ ID NO: 3, SEQ ID NO: 5, SEQ ID NO: 7, SEQ ID NO: 8, SEQ ID NO: 9, or SEQ ID NO: 10.

Specifically, the variant polypeptide of the present disclosure may have, comprise, consist of, or essentially consist of the amino acid sequence of SEQ ID NO: 3, SEQ ID NO: 5, SEQ ID NO: 7, SEQ ID NO: 8, SEQ ID NO: 9, or SEQ ID NO: 10, or an amino acid sequence having at least 70%, 80%, 85%, 90%, 95%, 96%, 97%, 98%, or 99% or more homology or identity thereto.

Examples include addition or deletion of sequences, naturally occurring mutations, silent mutations, or conservative substitutions in the N-terminus, C-terminus, and/or within the amino acid sequence that do not alter the function of the variant of the present disclosure.

As used herein, the term "conservative substitution" refers to the substitution of an amino acid with a different amino acid having similar structural and/or chemical properties. Such an amino acid substitution may generally occur based on similarity in polarity, charge, solubility, hydrophobicity, hydrophilicity, and/or amphipathic nature of residues. For example, positively charged (basic) amino acids include arginine, lysine, and histidine; negatively charged (acidic) amino acids include glutamic acid and aspartate; aromatic amino acids include phenylalanine, tryptophan, and tyrosine; and hydrophobic amino acids include alanine, valine, isoleucine, leucine, methionine, phenylalanine, tyrosine, and tryptophan. Additionally, amino acids may be classified into amino acids with electrically charged side chains and amino acids with uncharged side chains. The amino acids with electrically charged side chains include aspartate, glutamic acid, lysine, arginine, and histidine. The amino acids with uncharged side chains can be further classified into nonpolar amino acids and polar amino acids; nonpolar amino acids include glycine, alanine, valine, leucine, isoleucine, methionine, phenylalanine, tryptophan, and proline, and polar amino acids include serine, threonine, cysteine, tyrosine, asparagine, and glutamine. Typically, conservative substitution has little or no effect on the activity of the produced polypeptide. Typically, conservative substitution may have little or no effect on the activity of a protein or polypeptide.

Still another aspect of the present disclosure provides a polynucleotide encoding the variant polypeptide.

As used herein, the term "polynucleotide" refers to a polymer of nucleotides in which nucleotide monomers are linked in a long chain form by covalent bonds, which is a DNA or RNA strand of a certain length or longer, and more specifically, a polynucleotide fragment encoding the variant.

The polynucleotide encoding the variant polypeptide of the present disclosure may comprise any polynucleotide sequence without limitation, as long as the polynucleotide sequence encodes the variant polypeptide of the present disclosure. For example, the polynucleotide encoding the variant polypeptide of the present disclosure may be a polynucleotide sequence encoding an amino acid sequence of the variant polypeptide of the present disclosure, but is not limited thereto.

For example, the polynucleotide encoding the variant polypeptide of the present disclosure may comprise a nucleic acid sequence encoding the amino acid sequence set forth in SEQ ID NO: 3, SEQ ID NO: 5, SEQ ID NO: 7, SEQ ID NO: 8, SEQ ID NO: 9, or SEQ ID NO: 10. In one example of the present disclosure, the polynucleotide of the present disclosure may have or comprise the sequence of SEQ ID NO: 4 or SEQ ID NO: 6. Additionally, the polynucleotide of the present disclosure may consist of or essentially consist of the sequence of SEQ ID NO: 4 or SEQ ID NO: 6.

The polynucleotide of the present disclosure may have various modifications in the coding region within the scope that does not change the amino acid sequence of the variant of the present disclosure, in consideration of the codon degeneracy or codons preferred in the organism in which the variant of the present disclosure is to be expressed. Accordingly, it is apparent that a polynucleotide that can be translated, by codon degeneracy, into a polypeptide consisting of an amino acid sequence of the variant of the present disclosure or a polypeptide having a homology or identity thereto may also be included. Additionally, the polynucleotide of the present disclosure may be the sequence of SEQ ID NO: 4 or SEQ ID NO: 6, or a degenerate sequence thereof.

For example, the polynucleotide of the present disclosure may be a base sequence having 70%, 75%, 80%, 85%, 90%, 95%, 96%, 97%, 98%, or 99% or more and 100% or less homology or identity to the sequence of SEQ ID NO: 2, in which the codon encoding the amino acid corresponding to position 461 of SEQ ID NO: 2, which is glutamine, is substituted with a codon encoding an amino acid other than glutamine, for example, arginine, histidine, or glutamic acid; or in which the codon encoding the amino acid corresponding to position 461 of SEQ ID NO: 2, which is glutamine, is substituted with a codon encoding an amino acid other than glutamine, for example, arginine, histidine, or glutamic acid, and the codon encoding the amino acid corresponding to position 125 of SEQ ID NO: 2, which is alanine, is substituted with a codon encoding an amino acid other than alanine, for example, valine, but is not limited thereto. Additionally, it is apparent that a variant having a polynucleotide sequence in which part of the sequence is deleted, modified, substituted, conservatively substituted, or added is also included within the scope of the present disclosure, as long as the polynucleotide sequence has such homology or identity and encodes the amino acid sequence of the variant polypeptide of the present disclosure.

In another example, the polynucleotide of the present disclosure may have or comprise a nucleic acid sequence having 70% or more, 75% or more, 80% or more, 85% or more, 90% or more, 95% or more, 96% or more, 97% or more, 98% or more, and less than 100% homology or identity to the sequence of SEQ ID NO: 4 or SEQ ID NO: 6, or may consist of or essentially consist of a nucleic acid sequence having 70% or more, 75% or more, 80% or more, 85% or more, 90% or more, 95% or more, 96% or more, 97% or more, 98% or more, and less than 100% homology or identity to the sequence of SEQ ID NO: 4 or SEQ ID NO: 6, but is not limited thereto.

Additionally, the polynucleotide of the present disclosure may comprise, without limitation, a probe that can be prepared from a known gene sequence, for example, any polynucleotide sequence which may hybridize with a sequence complementary to all or part of the polynucleotide sequence of the present disclosure under stringent conditions.

The term "stringent conditions" refers to conditions that enable specific hybridization between polynucleotides. Such conditions are disclosed in detail in the literature (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, the stringent conditions may include conditions under which polynucleotides having high homology or identity, i.e., polynucleotides having 60% or more, 70% or more, 75% or more, 80% or more, 85% or more, 90% or more, 95% or more, 96% or more, 97% or more, 98% or more, or 99% or more homology or identity hybridize with each other, while polynucleotides having homology or identity lower than the above homology or identity do not hybridize with each other; or may include ordinary washing conditions of Southern hybridization, *i.e.,* washing once, specifically twice or three times, at a salt concentration and temperature corresponding to 60°C, 1×SSC, 0.1% SDS, specifically 60°C, 0.1×SSC, 0.1% SDS, and more specifically 68°C, 0.1×SSC, 0.1% SDS.

Hybridization requires that two nucleic acids have complementary sequences, although base mismatches are permissible depending on the stringency of the hybridization. The term "complementary" is used to describe a relationship between nucleotide bases that can hybridize with each other. For example, with respect to DNA, adenine is complementary to thymine, and cytosine is complementary to guanine. Accordingly, the polynucleotide of the present disclosure may also comprise an isolated nucleic acid fragment complementary to the entire sequence as well as a nucleic acid sequence substantially similar thereto.

Specifically, polynucleotides having a homology or identity to the polynucleotide of the present disclosure may be detected using hybridization conditions comprising a hybridization step at a Tₘ value of 55°C under the above-described conditions. Additionally, the Tₘ value may be 60°C, 63°C, or 65°C, but is not limited thereto, and may be appropriately adjusted by those skilled in the art depending on the purpose thereof.

The appropriate stringency for hybridizing the polynucleotides depends on the length and degree of complementarity of the polynucleotides, and these variables are well known in the art *(e.g.,* Sambrook *et al.,* supra).

As used herein, the term "homology" or "identity" refers to the degree of similarity between two given amino acid sequences or nucleotide sequences and may be expressed as a percentage. The terms "homology" and "identity" may often be used interchangeably.

The sequence homology or identity of conserved polynucleotides or polypeptides may be determined by the standard alignment algorithm, and default gap penalties established by the program used may be applied together. Substantially, homologous or identical sequences may generally hybridize with each other as a whole or in part under moderate or highly stringent conditions. It is apparent that the hybridization also includes hybridization with a polynucleotide containing usual codons or codons considering codon degeneracy in the polynucleotide.

Whether any two polynucleotide or polypeptide sequences have homology, similarity, or identity may be determined by, for example, a known computer algorithm such as the "FASTA" program (Pearson et al., (1988) [Proc. Natl. Acad. Sci. USA 85]: 2444) using default parameters. Alternatively, they may be determined by using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including the GCG program package (Devereux, J., et al., Nucleic Acids Research 12: 387 (1984)), BLASTP, BLASTN, FASTA (Atschul, S. F., [et al., J Molec Biol 215]: 403 (1990); Guide to Huge Computers, Martin J. Bishop, [ED.,] Academic Press, San Diego, 1994, and [CARILLO et al.](1988) SIAM J Applied Math 48: 1073). For example, the homology, similarity, or identity may be determined by using BLAST of the National Center for Biotechnology Information or ClustalW.

The homology, similarity, or identity between polynucleotides or polypeptides may be determined by comparing sequence information using the GAP computer program as in, for example, Needleman et al., (1970), J Mol Biol. 48:443, as described in Smith and Waterman, Adv. Appl. Math (1981) 2:482. In summary, the GAP program may be defined as the value obtained by dividing the number of similarly aligned symbols (*i.e*., nucleotides or amino acids) by the total number of the symbols in the shorter of the two sequences. The default parameters for the GAP program may include: (1) a binary comparison matrix (containing a value of 1 for identity and 0 for non- identity) and the weighted comparison matrix of Gribskov et al. (1986) Nucl. Acids Res. 14:6745 as disclosed in Schwartz and Dayhoff, eds., Atlas of Protein Sequence and Structure, National Biomedical Research Foundation, pp. 353-358 (1979) (or the EDNAFULL (EMBOSS version of NCBI NUC4.4) substitution matrix); (2) a penalty of 3.0 for each gap and an additional 0.10 penalty for each symbol in each gap (or a gap open penalty of 10, and a gap extension penalty of 0.5); and (3) no penalty for end gaps. Accordingly, the term "homology" or "identity" used herein refers to the relevance between sequences.

Still another aspect of the present disclosure provides a vector comprising the polynucleotide of the present disclosure. The vector may be an expression vector for expressing the polynucleotide in a microorganism, but is not limited thereto.

As used herein, the term "vector" may include a DNA construct comprising the nucleotide sequence of a polynucleotide encoding the target polypeptide, operably linked to an appropriate expression control region (or expression control sequence) so as to express the target polypeptide in an appropriate host. The expression regulatory sequence may include a promoter capable of initiating transcription, any operator sequence for regulating the transcription, a sequence encoding a suitable mRNA ribosome-binding site, and a sequence for regulating termination of transcription and translation. The vector, after transformation into a suitable microorganism, may be replicated or function independently of the genome of the host, or may be integrated into the genome itself.

The vector used herein is not particularly limited, and any vector known in the art may be used. Examples of commonly used vectors include plasmids, cosmids, viruses, and bacteriophages, either in their natural state or in a recombinant state. For example, as a phage vector or cosmid vector, pWE15, M13, MBL3, MBL4, IXII, ASHII, APII, t10, t11, Charon4A, Charon21A, *etc.* may be used; and as a plasmid vector, those based on pDZ, pBR, pUC, pBluescriptll, pGEM, pTZ, pCL, pET, *etc.* may be used. Specifically, pDZ, pDC, pDC24, pACYC177, pACYC184, pCL, pECCG117, pUC19, pBR322, pMW118, pCC1BAC vector, *etc*. may be used.

In one embodiment, a polynucleotide encoding a target polypeptide may be inserted into a chromosome through a vector for chromosomal insertion in a cell. The insertion of a polynucleotide into a chromosome may be performed by any method known in the art, such as homologous recombination, but is not limited thereto. A selection marker for confirming the chromosomal insertion may be further included. The selection marker is used to screen cells transformed with the vector, i.e., to confirm the insertion of the target nucleic acid molecule; markers that confer selectable phenotypes, such as drug resistance, nutrient requirements, resistance to cytotoxic agents, or expression of surface polypeptides, may be used. In an environment treated with a selective agent, only the cells that express the selection marker survive or exhibit a distinct phenotype, allowing for the selection of transformed cells.

As used herein, the term "transformation" means that a vector including a polynucleotide encoding a target polypeptide is introduced into a microorganism or microbe to enable expression of the polypeptide encoded by the polynucleotide in the microorganism. The transformed polynucleotide may include any polypeptide as long as it can be expressed in a microorganism, regardless of whether it is inserted and located into the chromosome of the microorganism or located outside of the chromosome. Additionally, the polynucleotide comprises DNA and/or RNA encoding a target polypeptide. The polynucleotide may be introduced in any form as long as the polynucleotide can be introduced and expressed in a microorganism. For example, the polynucleotide may be introduced into a microorganism in the form of an expression cassette, which is a gene construct including all factors required for self-expression. The expression cassette may comprise a promoter operably linked to the polynucleotide, a transcription termination signal, a ribosome binding site, and a translation termination signal. The expression cassette may be in the form of a self-replicating expression vector. Additionally, the polynucleotide may be introduced into the microorganism per se and operably linked to a sequence required for expression in the microorganism, but is not limited thereto.

Additionally, the term "operably linked" used herein refers to a functional linkage between a promoter sequence for initiating and mediating the transcription of the polynucleotide encoding the target protein of the present disclosure, and the polynucleotide sequence.

Still another aspect of the present disclosure provides a microorganism comprising the variant polypeptide or a polynucleotide encoding the variant polypeptide.

Specifically, the microorganism may comprise the variant polypeptide of the present disclosure, a polynucleotide encoding the variant polypeptide, or a vector comprising the polynucleotide.

In one specific embodiment, the microorganism of the present disclosure may be a microorganism having L-valine producing ability.

As used herein, the term "L-valine" refers to an L-amino acid having a chemical formula of (CH₃)₂CHCH(NH₂)COOH, which is an essential amino acid classified as a branched-chain amino acid along with L-leucine and L-isoleucine.

As used herein, the term "microorganism (or strain)" includes all wild-type microorganisms and microorganisms with natural or artificial genetic modification and refers to a microorganism in which a particular mechanism is weakened or enhanced due to the insertion of an exogenous gene, the enhancement or inactivation of an endogenous gene, or the like. The microorganism may be a microorganism comprising a genetic modification for the production of a target polypeptide, protein, or product. As used herein, the terms "microorganism", "strain", "host", and "host cell" have the same meaning and may be used interchangeably without limitation.

As used herein, the term "recombinant microorganism" refers to a microorganism that has been genetically modified to exhibit a different genotype and/or phenotype compared to a naturally occurring microorganism (e.g., when genetic modification affects how the base (nucleic acid) sequence is encoded in the microorganism), and may include all progeny or potential progeny of the microorganism. In the present disclosure, the terms "recombinant microorganism", "genetically modified microorganism", "recombinant host cell", "recombinant cell", and "recombinant strain" may be used interchangeably. The recombinant microorganism, for example, may express a gene that is not found in its natural (non-recombinant) form, may not express a gene that is expressed in its natural form, or may express a natural gene in a manner different from that in which it is expressed in its natural form.

The microorganism of the present disclosure may be a microorganism comprising one or more of the variant polypeptide of the present disclosure, the polynucleotide of the present disclosure, and a vector comprising the polynucleotide of the present disclosure; a microorganism modified to express the variant polypeptide of the present disclosure or the polynucleotide of the present disclosure; a recombinant microorganism expressing the variant polypeptide of the present disclosure or the polynucleotide of the present disclosure; or a recombinant microorganism having the activity of the variant polypeptide of the present disclosure, but is not limited thereto.

As used herein, the term "L-valine-producing microorganism" refers to a prokaryotic or eukaryotic microbial strain capable of producing L-valine within a live organism, and may include any microorganism in which L-valine producing ability has been imparted to a microorganism lacking L-valine producing ability as well as any microorganism that inherently possesses L-valine producing ability. The L-valine producing ability may be imparted or enhanced by strain improvement.

In one embodiment, the microorganism of the present disclosure may be a microorganism naturally possessing the variant polypeptide of the present disclosure or L-valine producing ability; or a microorganism in which the variant of the present disclosure or a polynucleotide encoding the same (or a vector comprising the polynucleotide) is introduced and/or L-valine producing ability is imparted to a parent strain without the variant polypeptide of the present disclosure or L-valine producing ability, but is not limited thereto.

In one embodiment, the microorganism of the present disclosure includes any microorganism comprising the sequence of the variant polypeptide of the present disclosure through mutations in the chromosomal gene encoding the variant polypeptide of the present disclosure and/or any microorganism comprising the variant polypeptide of the present disclosure through the introduction of a vector comprising a polynucleotide encoding the variant polypeptide of the present disclosure, but is not limited thereto.

As used herein, the term "non-modified microorganism (strain)" does not exclude a microorganism (strain) comprising mutations that may occur naturally, and may refer to a wild-type microorganism (strain) or a natural microorganism (strain) per se, or a microorganism (strain) before a trait is altered by genetic variation due to natural or artificial factors. In the present disclosure, the term "non-modified microorganism (strain)" may be used interchangeably with "microorganism (strain) before modification", "non-mutated microorganism (strain)", "parent strain", "parent microorganism", "wild-type microorganism (strain)", "reference microorganism (strain)", or "standard organism (strain)". The non-modified used herein may refer to a microorganism (strain) into which the variant polypeptide of the present disclosure has not been introduced or before being introduced, or microorganism (strain) comprising a wild-type polypeptide, but is not limited thereto. For example, the non-modified microorganism used herein may be a microorganism comprising a polypeptide consisting of SEQ ID NO: 1 or a polynucleotide consisting of SEQ ID NO: 2, but is not limited thereto.

In one example, the microorganism of the present disclosure includes any microorganism comprising the sequence of the variant polypeptide of the present disclosure by mutations in the chromosomal gene encoding a polypeptide comprising the parent sequence of the present disclosure and/or any microorganism comprising the variant polypeptide of the present disclosure through the introduction of a vector comprising a polynucleotide encoding the variant polypeptide of the present disclosure, but is not limited thereto.

For the purposes of the present disclosure, the microorganism of the present disclosure may include any microorganism that comprises the variant polypeptide of the present disclosure and is capable of producing the target L-valine. In one example, the microorganism of the present disclosure may be a microorganism with increased L-valine producing ability by transforming with a vector comprising a polynucleotide encoding the variant polypeptide of the present disclosure and expressing the variant polypeptide of the present disclosure, but is not limited thereto. In another example, the microorganism of the present disclosure may be a microorganism with increased L-valine producing ability by introducing a polynucleotide encoding the variant polypeptide of the present disclosure into a naturalwild-type microorganism or an L-valine-producing microorganism and thereby expressing the variant polypeptide of the present disclosure, but is not limited thereto. The microorganism with increased L-valine producing ability may be a genetically engineered microorganism or a recombinant microorganism, and the microorganism with increased L-valine producing ability may be a microorganism with increased L-valine producing ability compared to a natural wild-type microorganism or a non-modified microorganism (such as a microorganism expressing a polypeptide including wild-type polypeptides or parent sequences or a microorganism that does not express the variant polypeptide of the present disclosure), but is not limited thereto.

In one example, the microorganism having L-valine producing ability of the present disclosure may include any microorganism transformed with a vector to express the variant polypeptide of the present disclosure and to produce L-valine.

The microorganism of the present disclosure may include any microorganism capable of expressing the variant polypeptide of the present disclosure through various known methods, in addition to the introduction of the polynucleotide or vector.

In one example, the microorganism with increased L-valine producing ability of the present disclosure may be a microorganism with increased L-valine producing ability compared to a parent microorganism (parent strain) before modification or a non-modified microorganism (for example, a microorganism that expresses the wild-type polypeptide, a polypeptide comprising a parent sequence, or a polypeptide of SEQ ID NO: 1, or a microorganism that does not express the variant polypeptide of the present disclosure), but is not limited thereto. In one example, the non-modified strain, which is the reference strain for comparing the increase in L-valine producing ability, may be KCCM11201P strain (US 8465962 B), CJ7V strain (US 11180784 B2), or CJ8V strain (US 11180784 B2), but is not limited thereto.

In one example, the microorganism with increased L-valine producing ability of the present disclosure may be a microorganism with increased L-valine producing ability compared to a microorganism comprising the polypeptide of SEQ ID NO: 1 or a polynucleotide encoding the same, but is not limited thereto.

In another example, the microorganism with increased L-valine producing ability of the present disclosure may be a microorganism with increased L-valine producing ability compared to a microorganism comprising the polypeptide in which the amino acid corresponding to position 42 in SEQ ID NO: 1 is substituted with valine or a polynucleotide encoding the same.

In one example, the microorganism with increased L-valine producing ability may have L-valine producing ability increased by about 1% or more, specifically, about 1% or more, about 2% or more, about 3% or more, about 5% or more, about 10% or more, about 15% or more, about 16% or more, about 17% or more, about 18% or more, about 19% or more, about 20% or more, about 21% or more, about 22% or more, or about 23% or more (the upper limit is not particularly limited and may be, for example, about 200% or less, about 150% or less, about 100% or less, about 90% or less, about 80% or less, about 70% or less, about 65% or less, about 60% or less, about 55% or less, about 50% or less, about 45% or less, about 40% or less, about 35% or less, about 30% or less, or about 25% or less) compared to the L-valine producing ability of the parent strain before modification or non-modified microorganism. However, as long as the microorganism shows a positive increase in L-valine producing ability compared with the parent strain or non-modified microorganism, it is not limited thereto. In another example, the microorganism with increased L-valine producing ability may have an increase of about 1.01 times or more, about 1.02 times or more, about 1.03 times or more, about 1.05 times or more, about 1.1 times or more, about 1.15 times or more, about 1.16 times or more, about 1.17 times or more, about 1.18 times or more, about 1.19 times or more, about 1.20 times or more, about 1.21 times or more, about 1.22 times or more, or about 1.23 times or more (the upper limit is not particularly limited, for example, about 10 times or less, about 5 times or less, about 3 times or less, about 2 times or less, about 1.5 times or less, or about 1.3 times or less) compared to that of a parent strain before modification, that of a non-modified microorganism, or that of an L-threonine efflux protein non-modified microorganism, but is not limited thereto. The term "about" refers to a range encompassing ±0.5, ±0.4, ±0.3, ±0.2, ±0.1, *etc.,* including all numerical values in a range equal to or similar to the numerical value following the term "about", but the range is not limited thereto.

In a microorganism according to any one of the foregoing specific embodiments, the microorganism of the present disclosure may be a microorganism of the genus *Corynebacterium,* a microorganism of the genus *Escherichia,* a microorganism of the genus *Erwinia,* a microorganism of the genus *Serratia,* a microorganism of the genus *Providencia,* a microorganism of the genus *Pseudomonas,* a microorganism of the genus *Leptospira,* a microorganism of the genus *Salmonella,* a microorganism of the genus *Brevibacteria,* a microorganism of the genus *Hyphomonas,* a microorganism of the genus *Chromobacterium,* a microorganism of the genus *Norcadia,* or a microorganism belonging to fungi or yeast, and specifically be a microorganism of the genus *Corynebacterium,* but is not limited thereto.

In one embodiment of the present disclosure, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.* Specifically, the microorganism of the present disclosure may be a microorganism of the genus *Corynebacterium,* or more specifically *Corynebacterium glutamicum,* but is not limited thereto.

Meanwhile, the microorganism having L-valine producing ability of the present disclosure includes any natural wild-type microorganism *per se,* microorganism with increased L-valine producing ability by enhancing or weakening the activity of a gene related to L-valine production mechanism, or microorganism with increased L-valine producing ability by introducing or enhancing the activity of an exogenous gene.

Meanwhile, it is already known that microorganisms of the genus *Corynebacterium* are capable of producing L-valine; however, their production capacity is significantly low, and the genes involved in the production mechanism and the underlying principles of the mechanism have not yet been fully elucidated. Accordingly, the microorganism having L-valine producing ability of the present disclosure may include any natural wild-type microorganism, *i.e.,* a microorganism of the genus *Corynebacterium* with increased L-valine producing ability by enhancing or weakening the expression of a gene related to L-valine production mechanism, or a microorganism of the genus *Corynebacterium* with increased L-valine producing ability by introducing or enhancing an exogenous gene.

As used herein, the term "introduction" refers to the method of delivering a polynucleotide encoding the acetohydroxy acid synthase variant or a vector comprising thereof into a host cell. Such introduction can be readily achieved using a common method in the art. The common method includes CaCl₂ precipitation, the Hanahan method using the reducing agent dimethyl sulfoxide (DMSO) to the CaCl₂ method to increase the efficiency, electroporation, calcium phosphate precipitation, protoplast fusion, stirring using silicon carbide fibers, transformation using PEG, and transformation mediated by dextran sulfate, Lipofectamine, or dry/inhibitory conditions Methods for vector transformation are not limited to these examples, and any transformation or transfection methods commonly used in the art may be used without limitation. Additionally, the delivered polynucleotide may be introduced and located within the chromosome of the host cell or be located outside the chromosome, as long as it can be expressed within the host cell. Furthermore, the polynucleotide may be introduced into the host cell in any form, as long as it can be expressed within the host cell. For example, the nucleotide may be introduced into the host cell in the form of an expression cassette, which is a polynucleotide construct comprising all the necessary elements for self-expression, but the form is not limited thereto. The expression cassette generally includes a promoter operably linked to the open reading frame (hereinafter referred to as "ORF") of the gene, a transcription termination signal, a ribosome binding site, and a translation termination signal. The expression cassette may be in the form of a self-replicable expression vector. Further, the polynucleotide may be introduced into the host cell per se and be operably linked to the sequence necessary for expression in the host cell, but is not particularly limited thereto.

Still another aspect of the present disclosure provides a method for producing L-valine, comprising culturing the microorganism of the present disclosure in a medium.

Specifically, the method for producing L-valine of the present disclosure may comprise culturing, in a medium, a microorganism comprising: the variant of the present disclosure, the polynucleotide of the present disclosure, or the vector of the present disclosure, but is not limited thereto.

As used herein, the term "culturing" means growing the microorganism of the present disclosure under appropriately controlled environmental conditions. The culturing process of the present disclosure may be performed in a suitable medium known in the art under suitable culturing conditions known in the art. Such a culturing process may be readily adjusted and used by those skilled in the art according to the selected strain. Specifically, the culturing may be batch culturing, continuous culturing, and fed-batch culturing, but is not limited thereto.

As used herein, the term "medium" refers to a mixed substance containing nutrients required to culture the microorganism of the present disclosure as a main component, and the medium supplies nutrients, growth factors, *etc.,* including water, which are indispensable for survival and development. Specifically, any medium and culture conditions may be used for culturing the microorganism of the present disclosure without particular limitation, as long as the medium is used for the common culture of microorganisms. The microorganism of the present disclosure may be cultured in a common medium containing suitable carbon sources, nitrogen sources, phosphorus sources, inorganic compounds, amino acids, and/or vitamins, *etc*., while controlling the temperature, pH, *etc*. under aerobic conditions. For example, a culture medium for a strain of the genus *Corynebacterium* can be found in the document ["Manual of Methods for General Bacteriology" by the American Society for Bacteriology (Washington D.C., USA, 1981)].

In the present disclosure, the carbon sources include carbohydrates such as glucose, saccharose, lactose, fructose, sucrose, and maltose; sugar alcohols such as mannitol and sorbitol; organic acids such as pyruvic acid, lactic acid, and citric acid; amino acids such as glutamic acid, methionine, and lysine; glycerol; and propanediol. Additionally, natural organic nutrients such as starch hydrolysates, molasses, blackstrap molasses, rice bran, cassava, sugarcane bagasse, and corn steep liquor may be used. Specifically, carbohydrates such as glucose and sterilized pretreated molasses (*i.e.,* molasses converted into reducing sugars) may be used, and other various carbon sources in appropriate amounts may be used without limitation. These carbon sources may be used alone or in combination of two or more, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate, or organic nitrogen sources such as amino acids such as glutamic acid, methionine, and glutamine, peptone, NZ-amine, meat extracts, yeast extract, malt extract, corn steep liquor, casein hydrolysate, fish or decomposition products thereof, and skim soybean cake or decomposition products thereof may be used. These nitrogen sources may be used alone or in combination of two or more, but are not limited thereto.

As the phosphorus sources, monobasic potassium phosphate, dibasic potassium phosphate, or the corresponding sodium-containing salts may be used. As the inorganic compounds, sodium chloride, calcium chloride, iron chloride, magnesium sulfate, iron sulfate, manganese sulfate, calcium carbonate, *etc.* may be used. In addition, amino acids, vitamins, and/or suitable precursors, *etc.* may be included. These components or precursors may be added to the medium in a batchwise or continuous manner. However, the medium is not limited thereto.

Additionally, during the culturing of the microorganism of the present disclosure, compounds such as ammonium hydroxide, potassium hydroxide, ammonia, phosphoric acid, and sulfuric acid may be added to the medium in a suitable manner to adjust the pH of the medium. During the culturing, an antifoaming agent such as fatty acid polyglycol ester may be used to suppress foaming. To maintain an aerobic state of the medium, oxygen or oxygen-containing gas may be injected into the medium. To maintain an anaerobic or microaerobic state of the medium, no gas or nitrogen, hydrogen, or carbon dioxide gas may be injected. However, the culturing conditions are not limited thereto

In the culturing of the present disclosure, the culturing temperature may be maintained at 20°C to 45°C, specifically 25°C to 40°C, and the culturing may be performed for about 10 to 160 hours, but the culturing conditions are not limited thereto.

L-valine produced by the culturing of the present disclosure may be secreted into the medium or remain in cells.

In one specific embodiment, the method for producing L-valine of the present disclosure may further comprise preparing the microorganism of the present disclosure, preparing a medium for culturing the strain, or a combination thereof (in any order), for example, before culturing.

In one specific embodiment, the method for producing L-valine of the present disclosure may further comprise recovering L-valine from the medium according to the culturing (the medium in which culturing has been performed) or from the microorganism of the present disclosure. The recovering may be further included after the culturing.

The recovering may be collecting L-valine using suitable methods known in the art depending on the culturing method of the microorganism of the present disclosure, such as batch, continuous, or fed-batch culturing methods. For example, L-valine may be recovered from the medium or the microorganism using suitable methods known in the art, such as centrifugation, filtration, crystallization, treatment with protein precipitants (salting-out method), extraction, ultrasonic disruption, ultrafiltration, dialysis, various types of chromatography such as molecular sieve chromatography (gel filtration), adsorption chromatography, ion exchange chromatography, and affinity chromatography, HPLC, or a combination thereof.

Additionally, the method for producing L-valine of the present disclosure may further comprise purification. The purification may be performed using a suitable method known in the art. In one example, when the method for producing L-valine of the present disclosure comprises both the recovering and purification, the recovering and purification may be performed sequentially or non-sequentially, in any order, or they may be carried out simultaneously or integrated into a single step; however, the method is not limited thereto.

In the method of the present disclosure, the variant polypeptide, polynucleotide, L-valine, and the like are as described in the aspects above.

Still another aspect of the present disclosure provides a composition for producing L-valine, comprising: the variant polypeptide; a polynucleotide encoding the variant polypeptide; a vector comprising the polynucleotide; a microorganism comprising: the variant polypeptide, a polynucleotide encoding the variant polypeptide, or a vector comprising the polynucleotide; or a combination of two or more thereof.

The composition of the present disclosure may further comprise any suitable excipient commonly used in compositions for producing L-valine. Examples of such excipients include preservatives, wetting agents, dispersing agents, suspending agents, buffering agents, stabilizers, or isotonic agents, but the excipient is not limited thereto.

In one specific embodiment, the composition of the present disclosure may comprise each component in a microbially effective amount or an amount that can be suitably present in a composition for production.

In the composition of the present disclosure, the variant polypeptide, polynucleotide, L-valine, and the like are as described in the aspects above.

Another aspect of the present disclosure provides the use of the variant polypeptide of the present disclosure for L-valine production.

The variant polypeptide of the present disclosure, L-valine, and the like are as described in the aspects above.

Still another aspect of the present disclosure provides use of a microorganism comprising the variant polypeptide or a polynucleotide encoding the variant polypeptide for L-valine production.

The variant polypeptide of the present disclosure, polynucleotide, L-valine, and the like are as described in the aspects above.

Still another aspect of the present disclosure provides a method for increasing the L-valine producing ability of a microorganism, comprising modifying the microorganism to express the variant polypeptide of the present disclosure.

The variant polypeptide of the present disclosure, microorganism, L-valine, and the like are as described in the aspects above.

The modification may be applied using various methods well known in the art, for example, modifying the microorganism to express the variant of the present disclosure by transforming it with a vector comprising the polynucleotide of the present disclosure or a polynucleotide encoding the variant of the present disclosure, but is not limited thereto.

### [Mode for Carrying Out the Invention]

The present disclosure will be described in detail by way of Examples. However, these Examples are given for illustrative purposes only, and the scope of the present disclosure is not intended to be limited by these Examples. Meanwhile, the technical descriptions absent in the present disclosure may be sufficiently understood and readily practiced by those skilled in the art to which the present disclosure or related art pertains.

### Example 1. Selection of mutant strains with increased valine producing ability through artificial mutagenesis

### Example 1-1. Artificial mutagenesis using gamma ray exposure

To select mutant strains with increased valine producing ability, microbial mutations were induced using the following method.

For the induction of mutation, a physical method, namely gamma irradiation, was employed. High-energy gamma rays emitted from the Co-60 source flow along the chromosome and randomly trigger changes in the base sequence including base substitutions, deletions, and insertions. Since the level of sequence modification is proportional to the intensity of the gamma irradiation, high-dose gamma rays induce mutations at a high rate while simultaneously increasing the lethality of the strain. To secure a high-quality mutant library with a high diversity of mutation ratios, gamma irradiation conditions for a *Corynebacterium* strain subjected to gamma irradiation were first established. Using a high-dose gamma irradiator (Nordion) located at the Advanced Radiation Technology Institute of the Korea Atomic Energy Research Institute, 30 mL of seed culture medium with an absorbance value (562 nm) of 6.09 was irradiated with gamma rays at intensities of 0, 0.5, 1, 2, 3, 4, 5, 7.5, and 10 kGy/hr for 1 hour. After irradiation, 100 µL each of the irradiated solutions diluted with activation medium to 100, 10⁻¹, 10⁻², 10⁻³, and 10⁻⁴ dilution ratios were spread on agar plates. The plates were incubated statically at 30 °C for 48 hours, and the number of individual colonies formed was counted to calculate the lethality rate under each irradiation condition. The CFU/mL obtained at irradiation conditions of 0, 0.5, 1, 2, 3, 4, 5, 7.5, and 10 kGy/hr were 3.6 × 10⁸, 6.9 × 10⁷, 9.8 × 10⁶, 2.7 × 10⁵, 2.1 × 10³, 3.0 × 10¹, 0, 0, and 0, respectively. From these results, the corresponding lethality rates were calculated as 0%, 81.00467%, 97.31540%, 99.92589%, 99.99942%, 99.99999%, 100%, 100%, and 100%, confirming that a gamma irradiation condition of 4 kGy/hr was optimal for securing a high-quality mutant library with a high diversity of mutations based on the gamma irradiation on the *Corynebacterium* strain.

To obtain a microorganism with high L-valine producing ability, a gamma-irradiation-based mutant library was constructed using the KCCM11201P strain (US 8465962 B), and mutant strains with high L-valine producing ability were screened. For gamma irradiation of the KCCM11201P strain, the strain was cultured in a 500 mL flask containing 50 mL of seed medium in a shaking incubator at 30°C for 24 hours, thereby obtaining a culture with an absorbance of 7.84 at 562 nm. Subsequently, the culture was diluted using the seed medium to an absorbance of 6.10 at 562 nm. Using the high-dose gamma irradiator located at the Advanced Radiation Technology Institute of the Korea Atomic Energy Research Institute, 30 mL of the diluted solution was irradiated with gamma rays at an intensity of 4 kGy/hr for 1 hour. Subsequently, 100 µL of the irradiated solution was spread on each of 100 activation agar plates (90 × 15 mm Petri dishes). After static incubation of the Petri dishes at 30°C for 48 hours, approximately 40,000 individual colonies were obtained. To secure mutant stability, all individual colonies formed on the Petri dishes were collected, suspended in 20% glycerol solution, aliquoted into 1.5 mL microtubes (1 mL each), and stored in an ultra-low temperature freezer at -80°C.

### Example 1-2. Evaluation of fermentation potency of mutagenized strains and strain selection

To select mutant strains with increased L-valine producing ability compared to *Corynebacterium glutamicum* KCCM11201P, which was used as a parent strain, a fermentation potency test was performed on randomly mutagenized strains. For screening of mutants with high L-valine producing ability, the 20 % glycerol suspension stored at -80°C was thawed at room temperature and serially diluted with saline to 10⁻¹, 10⁻², 10⁻³, 10⁻⁴, and 10⁻⁵. Each dilution (100 µL) was spread onto activation medium and incubated statically at 30 °C for 24 hours to re-activate the cells as individual colonies. The reactivated colonies were transferred using a colony-picking device (Qpix 420, Molecular Devices Co.) into 96 deep-well plates containing 350 µL of production medium, which corresponds to 17 % of each well volume. In the same plates, the non-irradiated KCCM11201P strain was inoculated into four wells per plate as a control for screening mutants with high L-valine producing ability. The plates inoculated with both mutant and control strains were sealed with a gas-permeable seal (Mark 2, Azenta Co.) and cultured at 30°C, 1,000 rpm for 48 hours using a shaking incubator (Multitron, Infors-HT Co.). After 48 hours, the 96 deep-well plates were centrifuged using a centrifuge (Centrifuge 5810R, Eppendorf Co.) at 15°C and 4,000 rpm for 20 minutes. The supernatants from which the cells were removed were transferred to 96-well black polystyrene microplates (Corning Co.) using a liquid handler (Biomek i5, Beckman Coulter Co.) for NIR spectrometry analysis. Subsequently, NIR spectrometry was performed to obtain individual analysis spectra for each well, and among 10,304 mutant strains, 16 strains showing superior L-valine concentrations were primarily selected. These 16 strains were further cultured using the same method and under the same conditions as above, and the L-valine concentrations were analyzed.
[Nutrient Medium (pH 7.2)]
Glucose 10 g, Meat Juice 5 g, Polypeptone 10 g, Sodium Chloride 2.5 g, Yeast Extract 5 g, Agar 20 g, Urea 2 g (based on 1 L of distilled water)
[Production Medium (pH 7.0)]
Glucose 100 g, Ammonium Sulfate 40 g, Soybean Protein 2.5 g, Corn Steep Solids 5 g, Urea 3 g, Dipotassium Phosphate 1 g, Magnesium Sulfate Heptahydrate 0.5 g, Biotin 100 µg, Thiamine-HCl 1 mg, Calcium Pantothenate 2 mg, Nicotinamide 3 mg, Calcium Carbonate 30 g (based on 1 L of distilled water)

As a result, C7 strain (valine production: 4.2 g/L), in which the valine production is increased by two-fold compared to the parent strain KCCM11201P (valine production: 2.1 g/L), was selected.

### Example 2. Identification of mutation through gene sequencing

The main genes of the strain were sequenced and compared to those of the KCCM11201P strain. As a result, it was confirmed that the strain with increased valine producing ability included base sequence mutations at specific positions of the ORF region of the *ilvN* gene. Specifically, it was confirmed that the C7 strain identically included the A42V mutation in the parent strain KCCM11201P, along with a mutation introduced 461 bp upstream from the start codon of the *ilvN* gene. This mutation changed the original codon CAA (SEQ ID NO: 2) to CGA (SEQ ID NO: 4), resulting in the form (SEQ ID NO: 3) in which the amino acid at position 154 (glutamine) is substituted with arginine.

Hereinafter, the following Examples identify whether the application of Q154R mutation, which is inserted at a specific position in the *ilvN* gene, would affect the ability of the microorganism of the genus *Corynebacterium* to produce valine, a branched-chain amino acid.

### Example 3-1. Preparation of Corynebacterium glutamicum KCCM11201P strain introduced with IIvN mutation and evaluation of L-valine producing ability

For the insertion of an IIvN (Q154R) mutant into a *Corynebacterium glutamicum* KCCM11201P strain with A42V mutation, a vector comprising the target mutation was constructed. Specifically, the genomic DNA of the C7 strain was extracted using a G-spin Total DNA Extraction Mini Kit (Intron, Cat. No. 17045) according to the protocol provided in the kit, and PCR was performed using the genomic DNA as a template. The conditions for PCR were as follows: denaturation at 94°C for 5 minutes; 25 cycles of denaturation at 94°C for 30 seconds, annealing at 55°C for 30 seconds, and polymerization at 72°C for 150 seconds; and polymerization at 72°C for 7 minutes. A PCR product (hereinafter referred to as "mutated fragment 1") of 951 bp was obtained using SEQ ID NOS: 11 and 12.

The obtained mutated fragment 1 was ligated to a pDC24 vector (SEQ ID NO: 29) treated with the restriction enzyme Smal (New England Biolabs, Beverly, MA) by using an Infusion Cloning Kit (Takara Bio Inc., Otsu, Japan), followed by transformation into *Escherichia coli (E. coli)* DH5α. After transforming the constructed gene into E. *coli* DH5α, the transformed strains were selected in an LB medium containing kanamycin. DNA was obtained therefrom using a DNA-spin plasmid DNA purification kit (INTRON), which was used to construct a vector pDC24-ilvN(Q154R) comprising the mutated fragment 1.

**[Table 1]**

| Primer | Base sequence |
|---|---|
| SEQ ID NO: 11 | CCCGGG ATGGCTAATTCTGACGTCA |
| SEQ ID NO: 12 | CCCGGG TGTCTGGAGCCAGGAGCAT |

The pDC24-ilvN(Q154R) vector was transformed into *Corynebacterium glutamicum* KCCM11201P by homologous recombination on the chromosome (van der Rest et al., Appl Microbiol Biotechnol 52:541-545, 1999). Strains in which the vector was inserted into the chromosome by homologous sequence recombination were selected in a medium containing kanamycin (25 mg/L). A PCR using SEQ ID NOS: 11 and 12 was performed on the *Corynebacterium glutamicum* transformants that had undergone secondary recombination to identify a strain which includes the A42V mutation *(i.e.,* alanine was substituted with valine at position 42 in the amino acid sequence of SEQ ID NO: 1 within the ORF of the *ilvN* gene on the chromosome), which is included in the parent strain KCCM11201P, in addition to the substitution of glutamine with arginine at position 154 in the amino acid sequence of SEQ ID NO: 1 within the ORF of the *ilvN* gene on the chromosome. The recombinant strain was named *Corynebacterium glutamicum* KCCM11201P::ilvN(Q154R).

To compare the valine producing ability of the valine-producing strains *Corynebacterium glutamicum* KCCM11201P and KCCM11201P::ilvN(Q154R), flask evaluation was performed. Each strain was subcultured in a nutrient medium, inoculated into a 250 mL corner-baffled flask containing 25 mL of a production medium, and incubated with shaking at 200 rpm at 30°C for 72 hours. The concentration of L-valine was analyzed using HPLC, and the analyzed concentrations of L-valine are shown in Table 2.
[Nutrient Medium (pH 7.2)]
Glucose 10 g, Meat Juice 5 g, Polypeptone 10 g, Sodium Chloride 2.5 g, Yeast Extract 5 g, Agar 20 g, Urea 2 g (based on 1 L of distilled water)
[Production Medium (pH 7.0)]
Glucose 100 g, Ammonium Sulfate 40 g, Soybean Protein 2.5 g, Corn Steep Solids 5 g, Urea 3 g, Dipotassium Phosphate 1 g, Magnesium Sulfate Heptahydrate 0.5 g, Biotin 100 µg, Thiamine-HCl 1 mg, Calcium Pantothenate 2 mg, Nicotinamide 3 mg, Calcium Carbonate 30 g (based on 1 L of distilled water)

**[Table 2]**

| L-valine producing ability of KCCM11201P and KCCM11201P::ilvN(Q154R) | | | | |
|---|---|---|---|---|
| Strain | L-valine (g/L) | | | |
| | Batch 1 | Batch 2 | Batch 3 | Average |
| KCCM11201P | 2.7 | 2.9 | 2.7 | 2.7 |
| KCCM11201P::ilvN(Q154R) | 3.3 | 3.2 | 3.4 | 3.3 |

As a result, it was found that the KCCM11201P::ilvN(Q154R) strain exhibits L-valine producing ability increased by 22% compared to the KCCM11201P strain.

### Example 3-2. Preparation of Corynebacterium glutamicum KCCM11201P strains introduced with IIvN mutations and evaluation of L-valine producing ability - 2

To identify the effect of the *ilvN(Q154R)* mutation alone in the wild-type IIvN enzyme, a KCCM11201P strain in which glutamine was substituted with the wild-type arginine at position 154 in SEQ ID NO: 1 in the ORF region of the *ilvN* gene was prepared.

Specifically, the pDC24-*ilvN*(Q154R) vector was transformed into *Corynebacterium glutamicum* KCCM11201P by homologous recombination on the chromosome (van der Rest et al., Appl Microbiol Biotechnol 52:541-545, 1999). Strains in which the vector was inserted into the chromosome by homologous sequence recombination were selected in a medium containing kanamycin (25 mg/L). Subsequently, a PCR using SEQ ID NOS: 11 and 12 was performed on the *Corynebacterium glutamicum* transformants that had undergone secondary recombination to identify a strain in which the substitution of alanine with valine at position 42 in the amino acid sequence of SEQ ID NO: 1 within the ORF of the *ilvN* gene on the chromosome, which is a mutation included in the parent strain KCCM11201P, is restored to alanine in addition to the substitution of glutamine with arginine at position 154 in the amino acid sequence of SEQ ID NO: 1 within the ORF of the *ilvN* gene on the chromosome. The recombinant strain was named *Corynebacterium glutamicum* KCCM11201P::ilvN(V42A, Q154R). Additionally, to confirm the effect of Q154R only without the effect of restoring the A42V mutation in the *ilvN* gene in the KCCM11201P strain, a KCCM11201P::ilvN(V42A) strain with A42V mutation restoration using the same method was simultaneously selected as a control.

To compare the valine producing ability of KCCM11201P::ilvN(V42A) and KCCM11201P::ilvN(V42A, Q154R), flask evaluation was performed. Each strain was subcultured in a nutrient medium, inoculated into a 250 mL corner-baffled flask containing 25 mL of a production medium, and incubated with shaking at 200 rpm at 30°C for 72 hours. The concentration of L-valine was analyzed using HPLC, and the analyzed concentrations of L-valine are shown in Table 3.

**[Table 3]**

| L-valine producing ability of KCCM11201P::ilvN(V42A) and KCCM11201P::ilvN(V42A,Q154R) | | | | |
|---|---|---|---|---|
| Strain | L-valine (g/L) | | | |
| | Batch 1 | Batch 2 | Batch 3 | Average |
| KCCM11201P::ilvN(V42A) | 2.2 | 2.1 | 2.2 | 2.2 |
| KCCM11201P::ilvN(V42A, Q154R) | 2.7 | 2.6 | 2.7 | 2.7 |

As a result, it was found that the KCCM11201P::ilvN(V42A, Q154R) strain exhibits L-valine producing ability increased by 23% compared to that of the KCCM11201P::ilvN(V42A) strain.

### Example 4. Preparation of Corynebacterium glutamicum CJ7V strains introduced with IIvN mutations and evaluation of L-valine producing ability

In order to determine whether the increased L-valine producing ability could also be observed in other *Corynebacterium glutamicum* strains producing L-valine, a strain with improved L-valine producing ability was prepared by introducing a single mutation, *ilvN(A42V)* [Biotechnology and Bioprocess Engineering, June 2014, Volume 19, Issue 3, pp. 456-467], into the wild-type *Corynebacterium glutamicum* ATCC14067.

Specifically, the genomic DNA of the wild-type *Corynebacterium glutamicum* ATCC14067 strain was extracted using a G-spin Total DNA Extraction Mini Kit (Intron, Cat. No. 17045) according to the protocol provided in the kit, and PCR was performed using the genomic DNA as a template. To construct a vector for introducing the A42V mutation into the *ilvN* gene, two gene fragments (A and B) were obtained using the primer pair of SEQ ID NOS: 13 and 14 and the primer pair of SEQ ID NOS: 15 and 16, respectively. The conditions for PCR were as follows: denaturation at 94°C for 5 minutes; 25 cycles of denaturation at 94°C for 30 seconds, annealing at 55°C for 30 seconds, and polymerization at 72°C for 60 seconds; and polymerization at 72°C for 7 minutes.

As a result, polynucleotides of 528 bp and 509 bp were obtained for fragments A and B, respectively. Using the two fragments as templates, overlapping PCR was performed using the primer pair of SEQ ID NOs: 12 and 15 to obtain a 1,010 bp PCR product (hereinafter referred to as "mutated fragment 2").

The obtained mutated fragment 2 was digested with the restriction enzyme Smal (New England Biolabs, Beverly, MA) and ligated to the pDC24 vector treated with the same restriction enzyme using T4 ligase (New England Biolabs, Beverly, MA). After transforming the constructed gene into *E*. *coli* DH5α, the transformed strains were selected in an LB medium containing kanamycin. DNA was obtained therefrom using a DNA-spin plasmid DNA purification kit (INTRON). The vector constructed for the introduction of the A42V mutation into the *ilvN* gene was named pDC24-ilvN(A42V).

**[Table 4]**

| Primer | Base sequence |
|---|---|
| SEQ ID NO: 13 | cggggatcccccgggAGGACGGTACTCAAATACTAAACTTC |
| SEQ ID NO: 14 | TGCCGAGTGTTTCGGTCTTTACAGACACGAGGGACA |
| | CG |
| SEQ ID NO: 15 | TGTCTGTAAAGACCGAAACACTCGGCATCAA |
| SEQ ID NO: 16 | cggggatcccccgggGACAACTACATTATTATTATACCACA |

The pDC24-ilvN(A42V) vector was transformed into the wild-type *Corynebacterium glutamicum* ATCC14067 by homologous recombination on the chromosome (van der Rest et al., Appl Microbiol Biotechnol 52:541-545, 1999). Strains in which the vector was inserted into the chromosome by homologous sequence recombination were selected in a medium containing kanamycin (25 mg/L). PCR was performed on the *Corynebacterium glutamicum* transformant in which the second homologous recombination had been completed using the primers of SEQ ID NOs: 13 and 16 to amplify the gene fragment, and sequencing analysis was carried out to confirm the mutant strain in which the mutation had been inserted. The recombinant strain was named *Corynebacterium glutamicum* CJ7V.

Finally, a strain was prepared by transforming the *Corynebacterium glutamicum* CJ7V strain with the vector in the same manner as described in Example 3, and the resulting strain was named *Corynebacterium glutamicum* CJ7V::ilvN(Q154R). To compare the L-valine producing ability of the prepared strains, the strains were cultured in the same manner as in Example 3, and the concentration of L-valine was analyzed. The analyzed concentrations of L-valine are shown in Table 5 below.

**[Table 5]**

| L-valine producing ability of CJ7V, and CJ7V::ilvN(Q154R) | | | | |
|---|---|---|---|---|
| Strain | L-valine (g/L) | | | |
| | Batch 1 | Batch 2 | Batch 3 | Average |
| CJ7V | 2.2 | 2.1 | 2.2 | 2.2 |
| CJ7V::ilvN(Q154R) | 2.8 | 2.7 | 2.7 | 2.7 |

As a result, it was found that the CJ7V::ilvN(Q154R) strain exhibits L-valine producing ability increased by 23% compared to the CJ7V strain.

### Example 5. Preparation of Corynebacterium glutamicum CJ8V strains introduced with IIvN mutations and evaluation of L-valine producing ability

In order to determine whether the increased L-valine producing ability could also be observed in other *Corynebacterium glutamicum* strains producing L-valine, a strain with improved L-valine producing ability was prepared (KR 10-1947945 B1) by introducing a single mutation, *ilvN(A42V)* [Biotechnology and Bioprocess Engineering, June 2014, Volume 19, Issue 3, pp. 456-467], into *Corynebacterium glutamicum* ATCC13869.

Specifically, the pDC24-ilvN(A42V) vector constructed in Example 4 above was transformed into the wild-type *Corynebacterium glutamicum* ATCC13869 to induce homologous recombination on the chromosome (van der Rest et al., Appl Microbiol Biotechnol 52:541-545, 1999). Strains in which the vector was inserted into the chromosome by homologous sequence recombination were selected in a medium containing kanamycin (25 mg/L). Through PCR on the selected *Corynebacterium glutamicum* transformant using the primer set of SEQ ID NOS: 17 and 18, the gene fragment was amplified, and sequencing analysis was carried out to confirm that the mutation had been correctly inserted. The recombinant strain was named *Corynebacterium glutamicum* CJ8V. The primer sequences used in the present Example are as shown in Table 6 below.

**[Table 6]**

| Primer | Base sequence |
|---|---|
| SEQ ID NO: 17 | CCGCGTCACCAAAGCGGA |
| SEQ ID NO: 18 | TTAGATCTTGGCCGGAGCCA |

Finally, a strain was prepared by transforming the *Corynebacterium glutamicum* CJ8V with the vector in the same manner as described in Example 3, and the resulting strain was named *Corynebacterium glutamicum* CJ8V::ilvN(Q154R). To compare the L-valine producing ability of the prepared strains, the strains were cultured in the same manner as in Example 3, and the concentration of L-valine was analyzed. The analyzed concentrations of L-valine are shown in Table 7 below.

**[Table 7]**

| L-valine producing ability of CJ8V and CJ8V::ilvN(Q154R) | | | | |
|---|---|---|---|---|
| Strain | L-valine (g/L) | | | |
| | Batch 1 | Batch 2 | Batch 3 | Average |
| CJ8V | 1.9 | 1.8 | 1.9 | 1.9 |
| CJ8V::ilvN(Q154R) | 2.1 | 2.2 | 2.2 | 2.2 |

As a result, it was found that the CJ8V::ilvN(Q154R) strain exhibits L-valine producing ability increased by 16% compared to the CJ8V strain.

### Example 6. Preparation of KCCM11201P strains introduced with Q154X mutation and identification of valine producing ability

In order to confirm the effect of the mutation site at position 154 of IIvN, site-directed mutagenesis was performed using pDC24-ilvN(Q154R), which was used in Example 3-1, as a template, to substitute glutamine, the amino acid at position 154, with an amino acid other than arginine. Site-directed mutagenesis was performed using the following method.

**[Table 8]**

| Site-Directed Mutagenesis PCR composition | Unit (uL) |
|---|---|
| 10X pfu-X Buffer | 5 |
| 10mM dNTP Mix | 1 |
| pfu-X Polymerase | 1 |
| Mutagenic forward primer(5 pmol) | 2 |
| Mutagenic reverse primer(5 pmol) | 2 |
| pDC24-*ilvN*(Q154R) (template DNA, 200 ng/uL) | 1 |
| dH2O | 38 |
| Total | 50 |

**[Table 9]**

| Site-Directed Mutagenesis PCR cycle | | |
|---|---|---|
| Cycle | Temperature | Time |
| 1 | 95°C | 1 min |
| 18 | 95°C | 50 sec |
| | 60°C | 50 sec |
| | 68°C | 9 min |
| 1 | 68°C | 7 min |

To substitute the amino acid glutamine at position 154 of IIvN with an amino acid other than arginine, namely histidine (H) or glutamic acid (E), PCR was performed using each of the mutagenic primer sets listed in Table 10, with the PCR mixture prepared as shown in Table 8 and under the PCR conditions described in Table 9. After completion of the PCR, the amplified products were ligated using the Infusion Cloning Kit (Takara Bio Inc., Otsu, Japan), followed by transformation into *Escherichia coli* DH5α. Sequencing of the obtained pDC24_*ilvN* mutant plasmids confirmed that each plasmid had been substituted with the respective mutations listed in Table 10.

**[Table 10]**

| Mutagenic primer set for constructing plasmids with mutation at the amino acid position 154 in the IIvN amino acid sequence | | |
|---|---|---|
| *ilvN* plasmid mutant | SEQ ID NO | Sequence (5'-3') |
| pDC24_ilvN(Q154H) | 19 | CGAACTGATCcacTCCGGACAGA |
| | 20 | TCTGTCCGGAgtgGATCAGTTCG |
| *pDC24_ilvN(Q154E)* | 21 | CGAACTGATCgagTCCGGACAGA |
| | 22 | TCTGTCCGGActcGATCAGTTCG |

The vectors pDC24_*ilvN*(Q154H) and pDC24_*ilvN*(Q154E) constructed as shown in Table 10 were transformed into the KCCM11201P strain via electroporation. After a second crossover process, two types of strains with the chromosomally integrated mutant *ilvN* gene were obtained. The genetic modification was confirmed through PCR using the primers of SEQ ID NOS: 11 and 12, which respectively amplify the external regions of the upstream and downstream homologous recombination sites where the gene had been inserted, and by genome sequencing.

The thus-obtained transformed cells were named KCCM11201P::ilvN(Q154H) and KCCM11201P::ilvN(Q154E), respectively.

To compare the L-valine producing ability of the two strains prepared in Example 6 with the KCCM11201P::ilvN(Q154R) strain prepared in Example 3, the strains were cultured in the same manner as in Example 3, and the concentration of L-valine was analyzed. The analyzed concentrations of L-valine are shown in Table 11 below.

**[Table 11]**

| L-valine producing ability of strains in which the amino acid at position 154 in the IIvN amino acid sequence is mutated | | |
|---|---|---|
| | Strain Name | L-valine (g/L) |
| Control group | KCCM11201P | 2.7 |
| Experimental group | KCCM11201P::ilvN(Q154R) | 3.3 |
| | KCCM11201P::ilvN(Q154H) | 3.2 |
| | KCCM11201P::ilvN(Q154E) | 3.3 |

As a result, it was confirmed that the KCCM11201P::ilvN(Q154R) strain exhibits L-valine producing ability increased by 22% compared to that of KCCM11201P, the KCCM11201P::ilvN(Q154H) strain exhibits L-valine producing ability increased by 19% compared to that of the KCCM11201P, and KCCM11201P::ilvN(Q154E) strain exhibits L-valine producing ability increased by 22% compared to that of KCCM11201P.

As set forth above, those skilled in the art will be able to understand that the present disclosure may be embodied in other specific forms without departing from the technical spirit or essential characteristics thereof. In this regard, it should be understood that the foregoing examples are illustrative in all respects and are not to be construed as limiting. It should be understood that all changes or modifications derived from the definitions and the scopes of the claims and their equivalents fall within the scope of the present disclosure.

## Claims

1. A variant polypeptide in which an amino acid corresponding to position 154 in SEQ ID NO: 1 is substituted with a different amino acid.

2. The variant polypeptide of claim 1, wherein the amino acid corresponding to position 154 in SEQ ID NO: 1 is substituted with an electrically charged amino acid.

3. The variant polypeptide of claim 2, wherein the electrically charged amino acid is arginine, histidine, or glutamic acid.

4. The variant polypeptide of claim 1, wherein the variant polypeptide comprises any one amino acid sequence from amino acid sequences represented by Formula 1 to Formula 3 below:
[Formula 1]
EPFGIRELIX
[Formula 2]
PGKLRALLDVLEPFGIRELIX
[Formula 3] 
GINRITVVVDADELNIEQITKQLNKLIPVLKVVRLDEETTIARAIMLVKVSADST
NRPQIVDAANIFRARVVDVAPDSVVIESTGTPGKLRALLDVMEPFGIRELIX
 wherein in Formulas 1 to 3 above,
X is an electrically charged amino acid.

5. The variant polypeptide of claim 4, wherein the X is arginine, histidine, or glutamic acid.

6. The variant polypeptide of claim 1, wherein the variant polypeptide further comprises a substitution of an amino acid corresponding to position 42 in the amino acid sequence of SEQ ID NO: 1 with a different amino acid.

7. The variant polypeptide according to claim 6, wherein the different amino acid is valine.

8. The variant polypeptide of claim 6, wherein the variant polypeptide comprises an amino acid sequence of NLVSLVSV, LSVLVQDVDGIISRVSGMFTRRAFNLVSLVSV, or MANSDVTRHILSVLVQDVDGIISRVSGMFTRRAFNLVSLVSV.

9. The variant polypeptide of claim 1, wherein the variant polypeptide comprises an amino acid sequence having 70% or more and less than 100% identity to SEQ ID NO: 1.

10. The variant polypeptide of claim 1, wherein the variant polypeptide is a variant of an acetohydroxy acid synthase subunit.

11. The variant polypeptide of claim 1, wherein the variant polypeptide comprises an amino acid sequence of SEQ ID NO: 3, SEQ ID NO: 5, SEQ ID NO: 7, SEQ ID NO: 8, SEQ ID NO: 9, or SEQ ID NO: 10.

12. A polynucleotide encoding the variant polypeptide of any one of claims 1 to 11.

13. A microorganism comprising the variant polypeptide of any one of claims 1 to 11 or a polynucleotide encoding the variant polypeptide.

14. The microorganism of claim 13, wherein the microorganism has an increased L-valine-producing ability as compared to a microorganism comprising a polypeptide of SEQ ID NO: 1 or a polynucleotide encoding the same.

15. The microorganism of claim 13, wherein the microorganism is a microorganism of the genus *Corynebacterium.*

16. The microorganism of claim 15, wherein the microorganism of the genus *Corynebacterium* is *Corynebacterium glutamicum.*

17. A method for producing L-valine, comprising culturing the microorganism of claim 13 in a medium.

18. The method for producing L-valine according to claim 17, further comprising recovering L-valine from the cultured microorganism, a culture of the microorganism, a fermentation product of the microorganism, or the medium of the microorganism.

19. A composition for producing L-valine, comprising: the variant polypeptide of any one of claims 1 to 11; a polynucleotide encoding the variant polypeptide; a microorganism comprising the variant polypeptide or the polynucleotide encoding the variant polypeptide; a culture of the microorganism; or a combination of two or more thereof.

20. Use of the variant of any one of claims 1 to 11, or a microorganism comprising the variant polypeptide or a polynucleotide encoding the variant polypeptide for L-valine production.

21. A method for increasing the L-valine producing ability of a microorganism, comprising modifying a microorganism such that it expresses the variant polypeptide of any one of claims 1 to 11.
